# EUROPÄISCHE PATENTSCHRIFT

(11) **EP 0 644 780 B1**
(45) Veröffentlichungstag und Bekanntmachung des Hinweises auf die Patenterteilung: **19.03.1997**
(21) Anmeldenummer: 93912956.5
(22) Anmeldetag: 11.06.1993
(51) Int. Cl.: A61L 25/00, A61L 27/00, C04B 41/45

(54) **POLYMERGRANULAT ODER -FASERN UND HERSTELLUNGSVERFAHREN**
GRANULATE OR FIBROUS POLYMER AND METHOD OF PRODUCING IT
GRANULES OU FIBRES DE POLYMERE ET LEUR PROCEDE DE FABRICATION

(30) Priorität: 12.06.1992 DE 4219321
(43) Veröffentlichungstag der Anmeldung: 29.03.1995
(73) Patentinhaber: DRAENERT, Klaus, Dr.med., D-81545 München (DE)
(72) Erfinder: DRAENERT, Klaus, Dr.med., D-81545 München (DE)
(74) Vertreter: VOSSIUS & PARTNER
(86) Internationale Anmeldenummer: EP9301486
(87) Internationale Veröffentlichungsnummer: WO9325245

(56) Entgegenhaltungen:
- EP-A- 0 177 781
- EP-A- 0 276 836
- WO-A-86/03671
- WO-A-92/04924
- US-A- 3 766 000

## Beschreibung

Die Erfindung betrifft einen Werkstoff zur Verwendung als Knochenersatzwerkstoff oder als Verankerungsmittel von Endoprothesenkomponenten und ein Verfahren zu seiner Herstellung. Insbesondere betrifft die Erfindung ein Polymergranulat oder Polymerfasern und ein Verfahren zu deren Herstellung.

Ein Mehrkomponentenmaterial mit Zusätzen in Form von Füllerpartikeln, das als Implantationsmaterial verwendbar ist, ist beispielsweise aus der WO 92/04924 bekannt. Das Implantationsmaterial gemäß WO 92/04924 weist ein Polymer und/oder Copolymer sowie Füllerpartikel auf, die von dem Polymer- und/oder Copolymermaterial zumindest teilweise umschlossen werden. Das Implantationsmaterial wird beispielsweise dadurch hergestellt, daß die Füllerpartikel mit dem Perlpolymerisatpulver des Polymers und/oder Copolymers homogen durchmischt werden, das Gemisch danach verflüssigt und durch eine Düse in ein Fällungsbad ausgespritzt wird. Dabei werden Polymerkugeln erzeugt, in die die Füllerpartikel eingebettet sind. Die erzeugten Polymerkugeln werden anschließend gesiebt und weiterverarbeitet. Das im Rahmen der Weiterverarbeitung vollständig auspolymerisierte Material dient als Knochenersatzwerkstoff oder Verankerungsmittel von Endoprothesenkomponenten.

Der Erfindung liegt die Aufgabe zugrunde, einen Werkstoff zur Verwendung als Knochenersatzwerkstoff oder als Verankerungsmittel von Endoprothesenkomponenten bereitzustellen, welcher einfach, billig und in reproduzierbarer Weise hergestellt werden kann. Insbesondere liegt der Erfindung die Aufgabe zugrunde, einen derartigen Werkstoff bereitzustellen, der in nicht-kugelförmiger Gestalt vorliegt, um dadurch die Festigkeit des vollständig auspolymerisierten Knochenersatzwerkstoffes oder Verankerungsmittels zu erhöhen.

Diese Aufgabe wird durch die Erfindung gelöst. Die Erfindung geht dabei von dem Grundgedanken aus, ein Polymergranulat oder Polymerfasern bereitzustellen, wobei die einzelnen Granulatkörner oder Fasern Füllerpartikel, beispielsweise röntgenkontrastgebende Mittel, enthalten und die Polymerkomponente die Füllerpartikel zumindest teilweise, vorzugsweise weitgehend oder vollständig umschlossen hält. Die einzelnen Granulatkörner sollen dabei nicht kugelförmige Körper sein; polygonale, vieleckige, körnchenförmige, säulenförmige oder plattenförmige Körper oder Fasern sind besonders bevorzugt.

Der erfindungsgemäße Werkstoff ist vorzugsweise auf der Basis von organischen Polymeren, Acrylaten, Methacrylaten, Polymethylmethacrylaten und/oder Copolymeren derselben oder auf der Basis von Epoxidharzen oder ähnlichen Kunststoffen aufgebaut, die als Zweikomponenten-Kunststoffe verwendet werden können. Der nachstehend verwendete Begriff "Polymer" soll jeweils auch Copolymere mit umfassen.

Als Füller bzw. Füllerpartikel im Sinne der Erfindung können beispielsweise röntgenkontrastgebende Mittel, wie Zirkondioxid, Bariumsulfat, Silikat-Wismut-Verbindungen oder andere bekannte röntgenkontrastgebende Substanzen verwendet werden. Die Partikelgröße der Röntgenkontrastmittel beträgt vorzugsweise zwischen 1 und 15 µm, besonders bevorzugt etwa 5 µm. Es können außerdem auch andere Füllerpartikel verwendet werden, beispielsweise Füllerpartikel mit biomechanischer und/oder biologischer Wirkung. Beispiele für derartige Füllerpartikel sind Hydroxylapatit, Tricalciumphosphat, Calciumcarbonat oder andere Phosphat- oder Carbonatabkömmlinge, Metalle, wie Magnesium- oder Siliziumverbindungen, und/oder Aluminiumoxidkeramiken. Diese Füller können in Form eines fein-dispersen Pulvers mit einer Größe im µm-Bereich, beispielsweise 1 µm, vorliegen, sie können auch gröbere Partikel mit einer Größe von 20 µm bis zu mehreren mm, vorzugsweise bis etwa 200 - 250 µm sein. Während die groben Füller insbesondere zu einer Erhöhung der Härte bzw. Festigkeit des Endproduktes beitragen, bewirken die feindispersen Füller eine Aufrauhung der Oberfläche. Besonders bevorzugt ist deshalb eine Mischung von feindispersen und groben Füllern. Auch die genannten Füller können als röntgenkontrastgebende Substanz wirken.

Die Granulatkörner bzw. Granulatpartikel des Polymergranulats weisen vorzugsweise eine Größe von 1 bis 160 µm, besonders bevorzugt 40 bis 80 µm auf, wobei das Polymergranulat vorzugsweise ein Gemisch aus Partikeln verschiedener Größe innerhalb des vorstehenden Größenbereichs darstellt.

Die Fasern des erfindungsgemäßen Werkstoffes weisen vorzugsweise eine Dicke in derselben Größenordnung wie die Größe der Granulatkörner auf, d.h. 1 bis 160 µm, besonders bevorzugt 40 bis 80 µm. Die mechanischen Eigenschaften, insbesondere die Festigkeit des vollständig auspolymerisierten Knochenersatzwerkstoffes oder Verankerungsmittels erhöht sich insbesondere dann, wenn Fasern mit einer Länge von mehr als etwa 3 mm verwendet werden. Die bevorzugte Faserlänge beträgt somit etwa 3 bis 4 mm.

Dem erfindungsgemäßen Polymergranulat oder den erfindungsgemäßen Polymerfasern können zusätzlich ein oder mehrere Wirkstoffe zugesetzt, beigemengt oder in die Polymergranulatpartikel oder Fasern so eingebaut werden, daß sie wie die Füllerpartikel von der Polymerkomponente zumindest teilweise umschlossen werden. Geeignete Wirkstoffe sind beispielsweise Antibiotika, wie Gentamicin, Clindamicin, Lincomicin, Streptomicin oder Kombinationen hiervon oder ähnliche gut freisetzbare antibiotisch wirksame Substanzen. Als Wirkstoffe können auch Bone Morphogenetic Protein (BMP), Wachstumsfaktoren oder Wuchsstoffe auf der Basis des Wachstumshormons verwendet werden. Es ist auch möglich, der Polymerkomponente als Wirkstoff Metothrexat oder ein anderes geeignetes Zytostatikum beizumengen oder in die Polymergranulatpartikel oder -fasern einzubauen. Es können auch beliebige Kombinationen der genannten Wirkstoffe der Polymerkomponente zugesetzt oder in die Polymergranulatpartikel oder -fasern eingebaut werden.

Bei der Herstellung des erfindungsgemäßen Werkstoffes wird in einer Ausführungsform zunächst das Polymerpulver mit den Füllerpartikeln und gegebenenfalls zusätzlichen Wirkstoffen homogen vermischt. Als pulverförmiges Ausgangsmaterial wird dabei vorzugsweise handelsübliches Polymer-Perlpolymerisat ohne Monomer verwendet. Die Mischung erfolgt vorzugsweise im Verhältnis von 1 bis 15 g Füller auf 40 g Polymerpulver, vorzugsweise 8 bis 12 g Füller auf 40 g Polymerpulver, bzw. in größeren Mengen in dem vorgenannten Verhältnis.

Anschließend wird das Gemisch in einer Kammer erwärmt, bis es plastisch und extrudierfähig ist. Die Temperatur liegt hierbei unter derjenigen Temperatur, bei der eine vollständige Verflüssigung des Gemischs eintritt. Bei Verwendung handelsüblichen Polymer-Perlpolymerisats als Ausgangsmaterial beträgt die Temperatur bis zu 270°C, in der Regel weniger als etwa 250°C, vorzugsweise etwa 130 bis 220°C. Die Temperatur wird so eingestellt, daß die Polymerketten des Polymerpulvers nicht gecrackt werden und die Füllerpartikel und Wirkstoffe, wie die genannten Antibiotika, sich nicht zersetzen.

Anschließend wird das plastische Gemisch über eine Düse oder einen Extruder unter hohem Druck an Luft oder ein anderes Gas ausgepreßt. Hierzu können beispielsweise in der Kammer, in der das Gemisch erwärmt und in den plastischen Zustand überführt wird, mehrere Bohrungen mit einem Durchmesser von etwa 1 bis 5 mm, vorzugsweise etwa 2 bis 3 mm vorgesehen sein. Das Auspressen erfolgt bei einem Druck von mehreren bar, vorzugsweise etwa 5 bis 10 bar. Beim Auspressen oder Extrudieren der plastischen Masse durch jede Düse oder Bohrung entsteht eine längliche, wurstförmige Masse, die an Luft rasch erhärtet. Die gehärtete Masse wird anschließend in einer Mühle zu Polymergranulat vermahlen. Hierzu kann jede geeignete Mühle verwendet werden, bei der keine kugelförmigen, sondern granulatförmige Partikel gebildet werden, wie vorstehend erläutert, vorzugsweise in einer Größe von 1 bis 160 µm, besonders bevorzugt 40 bis 80 µm. Die Granulatpartikel sind nicht-kugelförmige Partikel, insbesondere vieleckige, langgestreckte oder säulenförmige Partikel. Das Granulat kann anschließend in der aus der WO 92/04924 bekannten Weisen weiterverarbeitet werden.

Das erfindungsgemäße Herstellungsverfahren kann auch dadurch modifiziert werden, daß das Polymerpulver zunächst getrennt von den Füllerpartikeln erwärmt und in einen plastischen und extrudierfähigen Zustand überführt wird. Die Füllerpartikel werden getrennt vom Polymerpulver ebenfalls erhitzt und werden dann unter hohem Druck in das plastische Polymer eingeschossen. Das Einschießen erfolgt vorzugsweise entweder kurz vor der Düse oder dem Extruder, durch die das Polymer ausgepreßt wird, oder auch innerhalb der Düse selbst oder kurz nach der Düse, solange die Masse noch plastisch ist.

Bei der Herstellung des erfindungsgemäßen Werkstoffes in Faserform wird im wesentlichen in der gleichen Weise vorgegangen, wie bei der Herstellung des granulatförmigen Werkstoffes. Das Polymerpulver kann zunächst mit den Füllerpartikeln vermischt und die Mischung erwärmt werden, Polymerpulver und Füllerpartikel können aber auch getrennt voneinander erwärmt und die Füllerpartikel erst kurz vor, während oder kurz nach dem Auspressen in das Polymer eingeschossen werden. Nach dem Erwärmen wird das plastisch verformbare Polymer bzw. das Gemisch aus Polymerpulver und Füllerpartikeln über mindestens eine Düse derart ausgepreßt, daß das Gemisch nach dem Auspressen als Faser aufgezogen und anschließend in die gewünschte Faserlänge geschnitten werden kann. Dies kann beispielsweise dadurch erreicht werden, daß die plastische Masse in ein temperiertes Wasserbad ausgepreßt und langsam abgekühlt wird. Hierbei bildet sich ein Endlosfaden bzw. eine Endlosfaser, die aufgewickelt und anschließend geschnitten werden kann.

Der erfindungsgemäße Werkstoff hat den Vorteil, daß die Füllerpartikel und gegebenenfalls Wirkstoffe außerordentlich homogen in der Polymerkomponente verteilt sind, so daß z.B. der Röntgenkontrast und die Wirkstoff-Freigabe sehr homogen ist. Die Granulat- oder Faserform der Polymerkomponente hat außerdem den Vorteil, daß bei der Weiterverarbeitung, wie der vollständigen Auspolymerisation des erfindungsgemäßen Werkstoffes, beispielsweise zu Knochenersatzwerkstoff oder Knochenzement, gegenüber der Verwendung kugelförmiger Polymerkomponenten eine erhöhte mechanische Festigkeit erzielt wird. Die Weiterverarbeitung kann einfach dadurch erfolgen, daß der erfindungsgemäße Werkstoff mit Monomer und gegebenenfalls einem Polymerisationsinitiator oder -beschleuniger versetzt und auspolymerisiert wird. Das Polymergranulat bzw. die Polymerfasern sind außerdem einfacher und reproduzierbarer herstellbar als mit dem in der WO 92/04924 beschriebenen Verfahren.

### Beispiel

Das Polymerpulver eines handelsüblichen Knochenzementes wird 12 Stunden lang im Wärmeofen bei 80°C getrocknet, anschließend in einen Extruderzylinder eingebracht und gleichmäßig auf 220°C erhitzt. Die sich dabei bildende plastische Masse wird anschließend über mehrere Düsen in dem Extruderzylinder in ein temperiertes Wasserbad ausgepreßt und langsam abgekühlt. Kurz vor dem Eintritt in die Düsen oder auch im Verlauf der Düsen, d.h. innerhalb der Düsen werden über feine Injektionsdüsen unter hohem Druck die vorher erwärmten Füllerpartikel eingeschossen. Als Material für die Füllerpartikel wird beispielsweise Zirkondioxid (ZrO₂) verwendet, das vorher bis zum rotglühenden Zustand erwärmt wurde. Auf diese Weise läßt sich eine homogene Verteilung der Füllerpartikel in der Polymerkomponente erzielen, so daß die Füllerpartikel von der Polymerkomponente zumindest teilweise umschlossen sind. Die im Wasserbad bei der Härtung der Masse gebildeten Fäden werden anschließend geschnitten und können gegebenenfalls in einer Mühle noch weiter gemahlen werden.

## Patentansprüche

1. Verfahren zur Herstellung eines Werkstoffs zur Verwendung als Knochenersatzwerkstoff oder als Verankerungsmittel von Endoprothesenkomponenten, wobei der Werkstoff ein Polymer und/oder Copolymer sowie Füllerpartikel enthält, wobei die Polymer- und/oder Copolymerkomponente die Füllerpartikel zumindest teilweise umschließt und der Werkstoff in Granulatform oder Faserform vorliegt, mit den folgenden Verfahrensschritten:
Herstellen eines plastischen Gemisches aus einem Polymer- und/oder Copolymerpulver und Füllerpartikeln bei einer Temperatur unterhalb derjenigen Temperatur, bei der eine vollständige Verflüssigung des Gemisches eintritt,
Auspressen der plastischen Masse über mindestens eine Düse, und
Erhärten der plastischen Masse und Vermahlen zu einem Granulat oder Aufziehen als Faser.

2. Verfahren nach Anspruch 1, wobei zunächst ein Polymer- und/oder Copolymerpulver mit Füllerpartikeln homogen vermischt wird und das Gemisch auf eine Temperatur erwärmt wird, bei der es zumindest plastisch ist.

3. Verfahren nach Anspruch 1, wobei die Füllerpartikel getrennt von dem Polymer- und/oder Copolymerpulver erhitzt werden und in heißem Zustand unter hohem Druck vor, während oder nach dem Auspressen in die plastische Polymer- und/oder Copolymerkomponente eingeschossen werden.

4. Verfahren nach einem der Ansprüche 1 bis 3, wobei die Faser anschließend geschnitten wird.

5. Verfahren nach einem der Ansprüche 1 bis 4, wobei erhitzte Füllerpartikel unter Druck in das Faserextrudat eingeschossen werden.

6. Verfahren nach einem der Ansprüche 1 bis 5, wobei als Polymer- und/oder Copolymerpulver-Ausgangsmaterial handelsübliches Polymer- und/oder Copolymer-Perlpolymerisat ohne Monomer verarbeitet wird und/oder wobei die Basis des Werkstoffes ein organisches Polymer, Acrylat, Methacrylat, Polymethylmethacrylat und/oder ein Copolymer desselben und/oder ein Epoxidharz oder ein ähnlicher Kunststoff ist, welcher als Zweikomponenten-Kunststoff verwendbar ist.

7. Verfahren nach einem der Ansprüche 1 bis 6, wobei Füllerpartikel und Polymer- und/oder Copolymerpulver im Verhältnis von 1 bis 15 g Füller auf 40 g Pulver, vorzugsweise 8 bis 12 g Füller auf 40 g Pulver, oder in einem entsprechenden Verhältnis in größerer Menge gemischt werden.

8. Verfahren nach einem der Ansprüche 1 bis 7, wobei das Gemisch auf eine Temperatur von bis zu 270°C, vorzugsweise weniger als 250°C, besonders bevorzugt 130 bis 220°C erwärmt wird und/oder wobei das Auspressen aus der Düse unter hohem Druck von mehreren bar, vorzugsweise mehr als 5 bar erfolgt.

9. Verfahren nach einem der Ansprüche 1 bis 8, wobei das Granulat ein Gemisch aus Partikeln einer Größe von 1 bis 160 µm, vorzugsweise 40 bis 80 µm ist, wobei die Granulatpartikel vorzugsweise polygonförmig, vieleckig oder säulenförmig sind und/oder eine andere nicht-kugelförmige Gestalt aufweisen.

10. Verfahren nach einem der Ansprühe 1 bis 9, wobei die Füllerpartikel röntgenkontrastgebende Mittel, wie Zirkondioxid, Wismut-Verbindungen und/oder Bariumsulfat sind, und/oder wobei als Füllerpartikel Hydroxylapatit, Tricalciumphosphat, Calciumcarbonat oder andere Phosphat- oder Carbonatabkömmlinge, Metalle, wie Magnesium- oder Siliziumverbindungen und/oder Aluminiumkeramiken verwendet werden, und/oder wobei die Füllerpartikel eine Partikelgröße zwischen 1 und 15 µm, vorzugsweise 3 bis 10 µm, besonders bevorzugt 5 µm aufweisen.

11. Verfahren nach einem der Ansprüche 1 bis 10, wobei Wirkstoffe in die Polymer- und/oder Copolymerkomponente eingebaut oder dem Granulat bzw. den Fasern zugesetzt werden, wobei die Wirkstoffe vorzugsweise Antibiotika sind, wobei als Antibiotika vorzugsweise Gentamicin, Clindamicin, Lincomicin, Streptomicin oder ähnliche gut freisetzbare antibiotisch wirksame Substanzen oder Kombinationen dieser Substanzen verwendet werden, oder wobei die Wirkstoffe Bone Morphogenetic Protein (BMP), Wachstumsfaktoren, Wuchsstoffe auf der Basis des Wachstumshormons und/oder Metothrexat oder ein anderes Zytostatikum sind.

12. Werkstoff zur Verwendung als Knochenersatzwerkstoff oder als Verankerungsmittel von Endoprothesenkomponenten, welcher ein Polymer und/oder Copolymer sowie Füllerpartikel enthält, wobei die Polymer- und/oder Copolymerkomponente die Füllerpartikel zumindest teilweise umschließt und die Ketten der Polymer- und/oder Copolymerkomponente nicht gecrackt sind, und wobei der Werkstoff in Granulatform oder Faserform vorliegt, herstellbar mit einem Verfahren nach einem der Ansprüche 1 bis 11.

## Claims

1. A method of producing a material to be used as a bone substitute material or a means for anchoring endoprosthesis components, wherein the material comprises a polymer and/or copolymer and filler particles, wherein the polymer and/or copolymer component encompasses the filler particles at least partially and wherein the material is in the form of granules or fibres, the method comprising the following steps:
preparing a plastic mixture of a polymer and/or copolymer powder and filler particles at a temperature below the temperature at which a complete liquefaction of the mixture occurs,
extruding the plastic mass via at least one nozzle, and
hardening the plastic mass and grinding it to obtain granules or drawing it to provide a fibre.

2. The method according to claim 1, wherein initially a polymer and/or copolymer powder is homogeneously mixed with filler particles and the mixture is heated to a temperature at which it is at least in a plastic state.

3. The method according to claim 1, wherein the filler particles are heated separately from the polymer and/or copolymer powder and injected into the plastic polymer and/or copolymer component in a hot state under high pressure before, during or after the extrusion.

4. The method according to any of claims 1 to 3, wherein the fibre is subsequently cut.

5. The method according to any of claims 1 to 4, wherein heated filler particles are injected into the extruded fibres under pressure.

6. The method according to any of claims 1 to 5, wherein conventional polymer and/or copolymer bead polymers without monomer are processed as the polymer and/or copolymer powder starting material, and/or wherein the material is based on an organic polymer, acrylate, methacrylate, polymethylmethacrylate and/or a copolymer thereof and/or an epoxy resin or a similar plastics material which can be used as a two-component plastics material.

7. The method according to any of claims 1 to 6, wherein the filler particles and the polymer and/or copolymer powder are mixed at a ratio of 1 - 15 g of filler to 40 g of powder, preferably 8 - 12 g of filler to 40 g of powder, or in a corresponding ratio in a larger quantity.

8. The method according to any of claims 1 to 7, wherein the mixture is heated to a temperature of up to 270°C, preferably less than 250°C, most preferably 130 to 220°C, and/or wherein the extrusion through the nozzle is effected under high pressure of several bar, preferably more than 5 bar.

9. The method according to any of claims 1 to 8, wherein the granules are a mixture of particles having a size of 1 to 160 µm, preferably 40 to 80 µm, wherein the granule particles are preferably polygonal, many-cornered or columnar and/or have another non-spherical shape.

10. The method according to any of claims 1 to 9, wherein the filler particles are radiographic contrast agents, such as zirconium dioxide, bismuth compounds and/or barium sulfate, and/or wherein hydroxylapatite, tricalcium phosphate, calcium carbonate or other phosphate or carbonate derivatives, metals, such as magnesium or silicon compounds and/or aluminum ceramics are used as the filler particles, and/or wherein the filler particles have a particle size of between 1 and 15 µm, preferably 3 to 10 µm, most preferably 5 µm.

11. The method according to any of claims 1 to 10, wherein active ingredients are incorporated into the polymer and/or copolymer component or added to the granules or fibres, wherein the active ingredients are preferably antibiotics, wherein preferably gentamicin, clindamicin, lincomicin, streptomicin or similar antibiotic substances having a good releasability or combinations of these substances are used as the antibiotics, or wherein the active ingredients are bone morphogenetic protein (BMP), growth factors, growth-promoting substances on the basis of the growth hormone and/or metothrexate or another cytostatic agent.

12. A material to be used as a bone substitute material or a means for anchoring endoprosthesis components, which comprises a polymer and/or copolymer and filler particles, wherein the polymer and/or copolymer component at least partially encompasses the filler particles, and the chains of the polymer and/or copolymer component are not cracked, and wherein the material is in the form of granules or fibers, the material being producible by means of a method according to any of claims 1 to 11.

## Revendications

1. Procédé de préparation d'un matériau à utiliser comme matériau de substitution osseux ou comme produit d'ancrage de composants d'endoprothèses, dans lequel le matériau contient un polymère et/ou copolymère ainsi que des particules de charge, le composant polymère et/ou copolymère entourant au moins partiellement les particules de charge et le matériau se présentant sous la forme d'un produit de granulation ou de fibres, ce procédé comportant les étapes suivantes :
une préparation d'un mélange plastique à partir d'une poudre de polymère et/ou de copolymère et de particules de charge, à une température en dessous de la température à laquelle il se produit une fluidification complète du mélange,
un pressage de la masse plastique par l'intermédiaire d'au moins une filière, et
un durcissage de la masse plastique et un broyage en un produit de granulation ou un étirage sous la forme de fibres.

2. Procédé suivant la revendication 1, caractérisé en ce qu'on mélange tout d'abord une poudre de polymère et/ou de copolymère de manière homogène avec des particules de charge et en ce qu'on chauffe le mélange à une température à laquelle il est au moins plastique.

3. Procédé suivant la revendication 1, caractérisé en ce que les particules de charge sont chauffées séparément de la poudre de polymère et/ou de copolymère et en ce qu'elles sont injectées à l'état chaud, sous haute pression, dans le composant polymère et/ou copolymère plastique avant, pendant ou après le pressage.

4. Procédé suivant l'une des revendications 1 à 3, caractérisé les fibres sont ultérieurement coupées.

5. Procédé suivant l'une des revendications 1 à 4, caractérisé en ce que des particules de charge chauffées sont injectées sous pression dans le produit d'extrusion en forme de fibres.

6. Procédé suivant l'une des revendications 1 à 5, caractérisé en ce que, comme matière de départ de poudre de polymère et/ou de copolymère, on traite un produit de polymérisation en perles polymère et/ou copolymère courant dans le commerce, sans monomère, et/ou en ce que la base du matériau est un polymère organique, de l'acrylate, du méthacrylate, du polyméthacrylate de méthyle et/ou un copolymère de celui-ci et/ou une résine époxy ou une matière synthétique analogue, qui est utilisable sous la forme d'une matière synthétique à deux composants.

7. Procédé suivant l'une des revendications 1 à 6, caractérisé en ce que les particules de charge et la poudre de polymère et/ou de copolymère sont mélangées dans un rapport de 1 à 15 g de charge pour 40 g de poudre, de préférence de 8 à 12 g de charge pour 40 g de poudre, ou dans un rapport correspondant en des quantités plus grandes.

8. Procédé suivant l'une des revendications 1 à 7, caractérisé en ce que le mélange est chauffé à une température allant jusqu'à 270°C, de préférence inférieure à 250°C, particulièrement avantageusement de 130 à 220°C, et/ou en ce que le pressage hors de la filière s'effectue sous une haute pression de plusieurs bars, de préférence de plus de 5 bars.

9. Procédé suivant l'une des revendications 1 à 8, caractérisé en ce que le produit de granulation est un mélange de particules d'une dimension de 1 à 160 µm, de préférence de 40 à 80 µm, les particules du produit de granulation présentant une configuration de préférence de forme polygonale, à plusieurs angles ou de colonne et/ou une autre configuration non sphérique.

10. Procédé suivant l'une des revendications 1 à 9, caractérisé en ce que les particules de charge sont des produits de contraste aux rayons X, comme du dioxyde de zirconium, des composés de bismuth et/ou du sulfate de baryum, et/ou en ce que, comme particules de charge, on utilise de l'hydroxylapatite, du phosphate tricalcique, du carbonate de calcium ou d'autres dérivés phosphatés ou carbonatés, des métaux, tels que des composés du magnésium ou du silicium et/ou des matières céramiques à base d'aluminium, et/ou en ce que les particules de charge présentent une dimension de particule comprise entre 1 et 15 µm, de préférence de 3 à 10 µm, particulièrement avantageusement de 5 µm.

11. Procédé suivant l'une des revendications 1 à 10, caractérisé en ce que des substances actives sont incorporées dans le composant polymère et/ou copolymère ou ajoutées au produit de granulation ou respectivement aux fibres, en ce que les substances actives sont de préférence des antibiotiques, de la gentamycine, de la clindamycine, de la lincomycine, de la streptomycine ou des substances antibiotiquement actives analogues, bien libérables, ou des combinaisons de ces substances étant de préférence utilisées comme antibiotiques, ou en ce que les substances actives sont une protéine morphogénétique de l'os (BMP), des facteurs de croissance, des substances de croissance à base de l'hormone de croissance et/ou du métothrexate ou un autre cytostatique.

12. Matériau à utiliser comme matériau de substitution osseux ou comme produit d'ancrage de composants d'endoprothèses, qui contient un polymère et/ou copolymère ainsi que des particules de charge, matériau dans lequel le composant polymère et/ou copolymère entoure au moins partiellement les particules de charge, les chaînes du composant polymère et/ou copolymère n'étant pas craquées, et dans lequel le matériau se présente sous la forme d'un produit de granulation ou de fibres, ce matériau étant préparable par un procédé suivant l'une des revendications 1 à 11.
